# EUROPEAN PATENT APPLICATION

(11) **EP 3 882 361 A1**
(43) Date of publication of application: **22.09.2021**
(21) Application number: 20382213.5
(22) Date of filing: 20.03.2020
(51) Int. Cl.: C12Q 1/683, G01N 33/558

(54) **LATERAL FLOW ASSAY FOR DETECTING THE PRESENCE OF CORONAVIRUS IN A BIOLOGICAL SAMPLE**

(71) Applicant: SOMAprobes SL, 20019 San Sebastián (ES)
(72) Inventor: Hernández Hincapié, Frank J., 581 83 Sweden (ES)
(74) Representative: Hoffmann Eitle

(57) **Abstract**

The present invention pertains to the medical field, in particular, to the use of lateral flow assays for detecting the presence of coronavirus in a biological sample.

## Description

### Technical field of the invention

The present invention pertains to the medical field, in particular, to the use of lateral flow assays for detecting the presence of coronavirus in a biological sample.

### Background of the invention

The SARS-CoV-2 Coronavirus pandemic represents a global challenge for all healthcare systems, since it is estimated that 60-70% of the population worldwide will be infected. The magnitude of this pandemic that quickly spread all over the world, makes it incompatible or almost impossible to provide a diagnostic test by the gold standard reverse transcript PCR (RT-PCR) for all the patients that will need it. Under these circumstances, innovative rapid diagnostic strategies for Coronavirus are fundamental for successful outbreak containment. This increased prevalence of Coronavirus not only influences the mortality and morbidity but also possesses a significant financial burden to the patient and the society, collapsing the healthcare services. Clinical experts, as well as local and global healthcare organisations highlight the urgent need for easy-to-use solutions that allow rapid and robust detection of Coronavirus to increase adequate management of the disease. **Currently, there is no Point-of-Care diagnostic tool available on the market capable of identifying Coronavirus in a specific manner in 25 min.** In fact, the detection of Coronavirus infections with the conventional tests (RT-PCR) takes more than 2 hours and in some cases even days, depending of the geographical location. Since the beginning of 2020, Point-of-Care devices based on the generic detection of IgM and IgG have been developed, however these methods are non-specific reporting significant number of false positives.

This underlines the obvious need for an accurate and faster diagnostic method for Coronavirus, especially for those patients asymptomatic or mildly symptomatic that were exposed or in contact with a patient diagnosed with the virus.

### Summary of the invention

The present invention provides for a rapid and sensitive molecular approach for the diagnosis of coronavirus infections, in particular for the diagnosis of SARS CoV-2 Coronavirus. In this sense, the instant invention intents to revolutionise the identification of Coronavirus infections with a strip-based test capable of rapidely detecting Coronavirus with high specificity and sensitivity. This diagnostic test is based on lateral flow assay (LFA) strips which feature its proprietary targeted approach based on the specific cleavage of nucleic acid substrates by the nsp15 endonuclease produced exclusively by Coronaviruses.

For that purpose, in a first aspect, the present invention provides for an *in vitro* use in a lateral flow assay of a polynucleotide sequence susceptible of being cleaved by the nsp15 endonuclease derived from coronovirus, wherein the polynucleotide sequence (also named "Substrate") is characterized by comprising a capture tag and a reporter molecule at each end of the sequence, for detecting the presence of coronavirus, in particular SARS CoV-2 Coronavirus, in a biological sample.

Preferably, said polynucleotide sequence is selected from the list consisting of SEQ ID No 1-34 below:

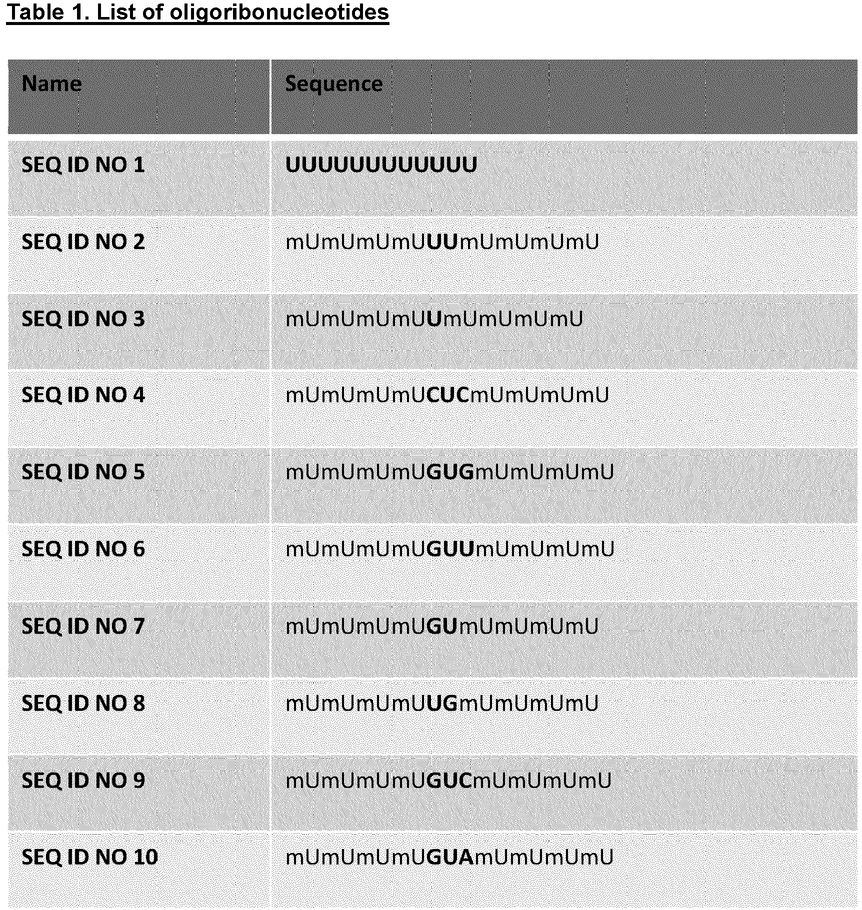

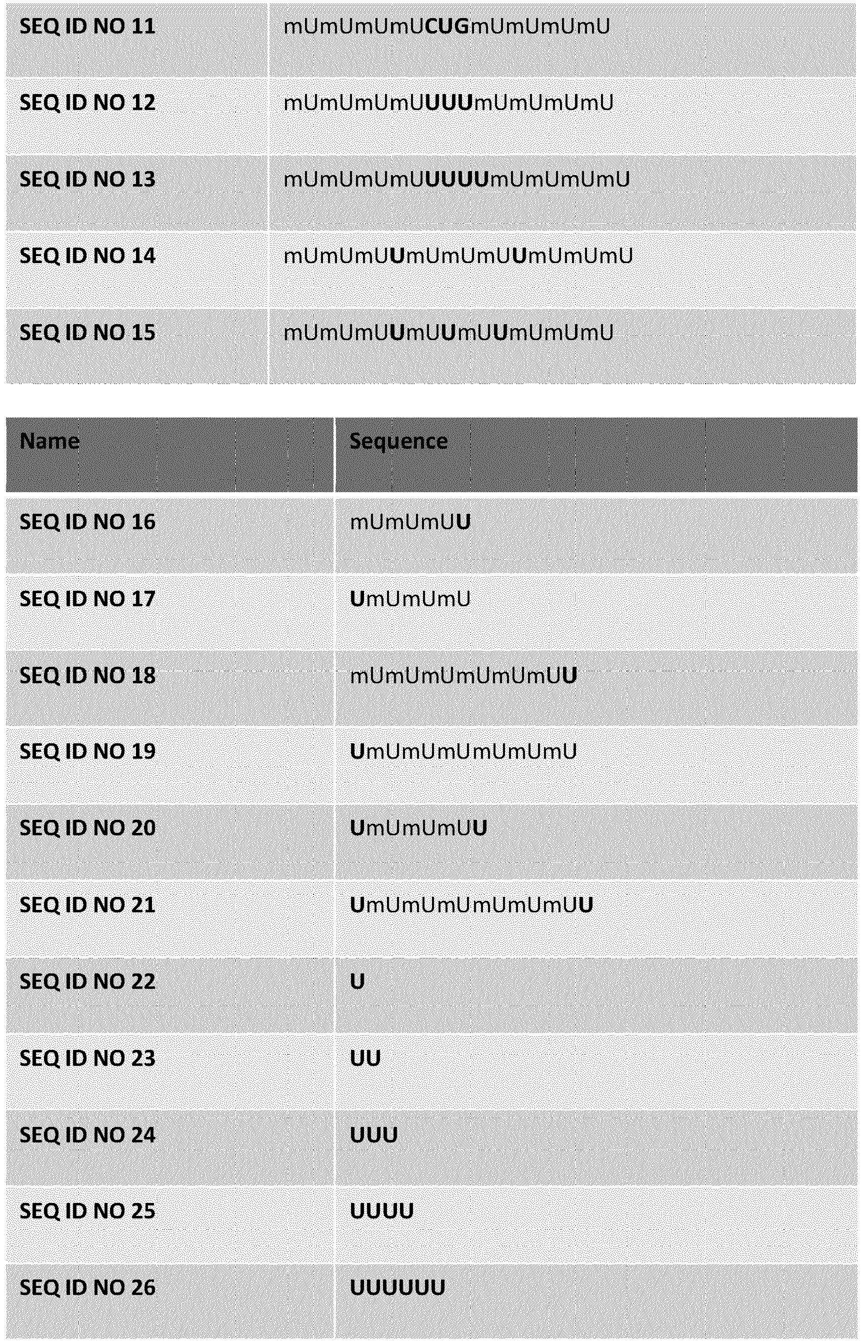

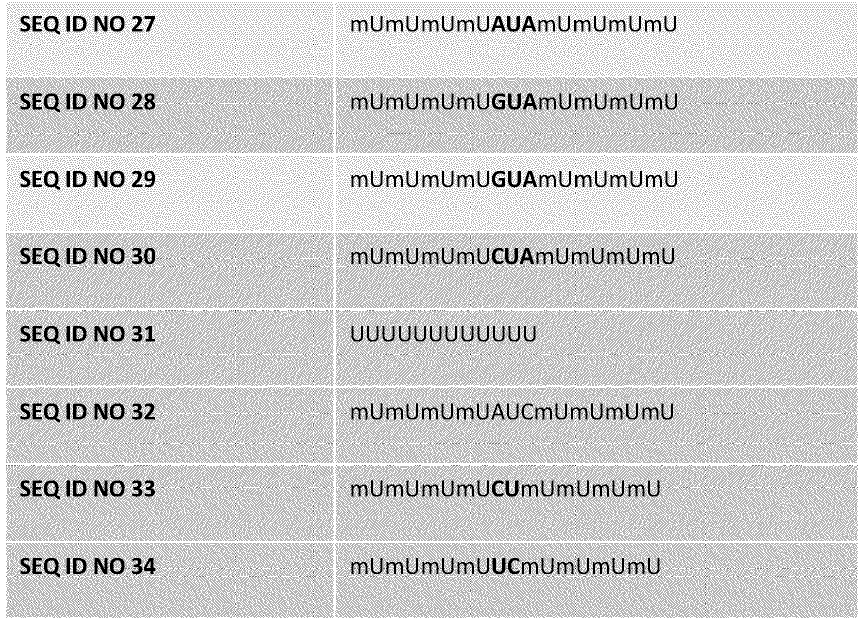

| | |
|---|---|
| Upper case = | RNA |
| mU= | 2'-O-Methyl modification |

It is important to note that, in a further embodiment, said polynucleotide sequence is selected from any of the list consisting of SEQ ID No 1-34, wherein mU has been replaced by _{LNA}U (Locked nucleic acid) (hereinafter LNA modified 1-34 sequences).

Preferably, the said capture tag is selected from the list consisting of **digoxigenin**:3-[(3*S*,5*R*,8*R*,9*S*,10*S*,12*R*,13*S*,14*S*,17*R*)-3,12,14-trihydroxy-10,13-dimethyl 1,2,3,4,5,6,7,8,9,11,12,15,16,17--tetradecahydrocyclopenta[a]phenanthren-17-yl]-2*H*-furan-5-one, **Biotin**: 5-[(3a*S*,4*S*,6a*R*)-2-oxohexahydro-1*H*-thieno[3,4-*d*]imidazol-4-yl]pentanoic acid, **ACA:** (6S,7S)-3-(acetoxymethyl)-7-amino-8-oxo-5-thia-1-azabicyclo[4.2.0]oct-2-ene-2-carboxylic acid **FAM:** 3',6'-dihydroxy-1-oxospiro[2-benzofuran-3,9'-xanthene]-5-carboxylic acid, **FITC:** 3',6'-dihydroxy-6-isothiocyanatospiro[2-benzofuran-3,9'-xanthene]-1-one, **Texas red:** 5-(Chlorosulfonyl)-2-(2,3,6,7,12,13,16,17-octahydro-1H,5H,11H,15H-pyrido[3,2,1-ij]quinolizino[1',9':6,7,8]chromeno [2,3-f]quinolin-4-ium-9-yl) Benzenesulfonate, **TAMRA:** 2-[3-(dimethylamino)-6-dimethylazaniumylidenexanthen-9-yl]benzoate or any other small antigen.

Preferably, the said reporter molecule is selected from the list consisting of latex and gold nanoparticles, carbon particles or any other colorimetric reporter molecule. More preferably the said reporter molecule is linked to the polynucleotide by using an antibody having specificity against the capture tag used to modify the polynucleotide probe.

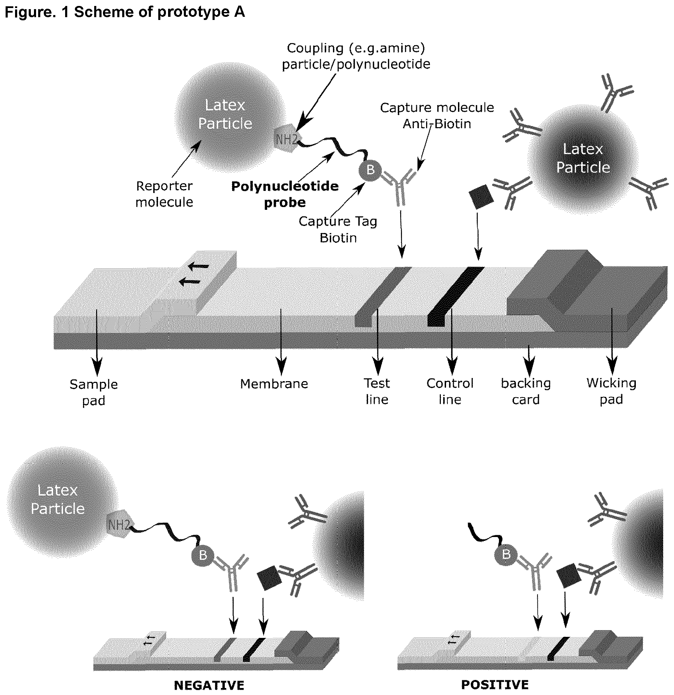

A second aspect of the present invention provides for a system that comprises:
a. The polynucleotide sequence as defined in the previous aspect;
b. A support suitable for lateral flow that comprises:
   b1. A backing card; and
   b2. A sample pad, a membrane and a wicking pad, all of them on top of the backing pad, wherein the membrane is located after the sample pad so that the sample flows from the sample pad to the membrane, wherein the membrane comprises a test line which in turn comprises molecules immobilized in the test line for capturing the capture tag of the polynucleotide sequence, and wherein the wicking pad is located at the end of the membrane (see Figure 1).

In a preferred embodiment of the second aspect of the invention, the membrane further comprises a control line.

A third aspect of the present invention provides for an *in vitro* use in a lateral flow assay of a polynucleotide sequence susceptible of being cleaved by the nsp15 endonuclease derived from coronovirus, wherein the polynucleotide sequence is characterized by comprising two capture tags at each end of the sequence, wherein each capture tag is different from the other and wherein one of the capture tags is capable of binding a reporter molecule, for detecting the presence of coronavirus, in particular SARS CoV-2 Coronavirus, in a biological sample..

In a preferred embodiment of the third aspect of the invention, the polynucleotide sequence is selected from the list consisting of SEQ ID No 1-34 or the LNA modified 1-34 sequences.

In another preferred embodiment of the third aspect of the invention, the capture tags are selected from the list consisting of BIOTIN, FITC, FAM, DIGOXIGENIN, DNP (DINITROPHENYL), TEXAS RED, TAMRA or any other small antigen.

In another preferred embodiment of the third aspect of the invention, the reporter molecule is selected from the list consisting of latex and gold nanoparticles, carbon particles or any other colorimetric reporter molecule.

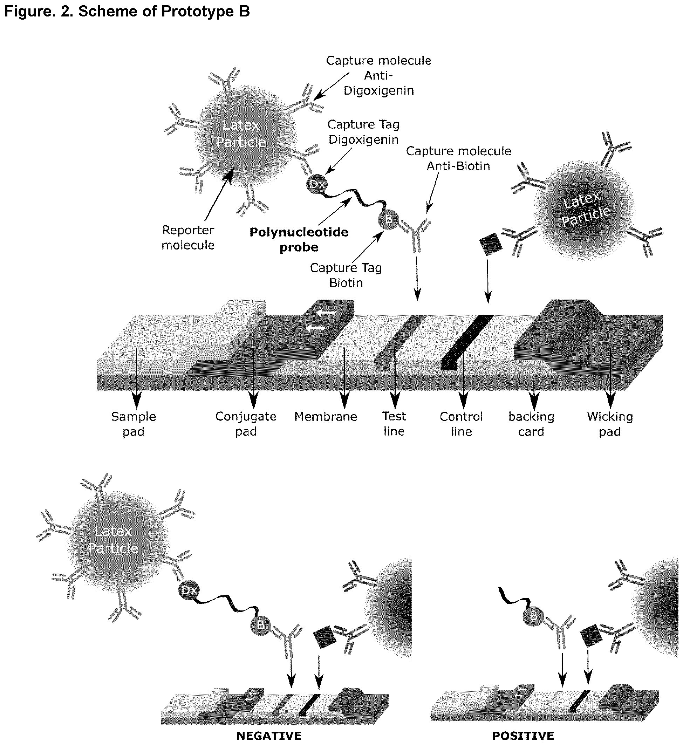

A fourth aspect of the invention provides for a system that comprises:
a. The polynucleotide sequence as defined in the third aspect of the invention, wherein one of the capture molecules is capable of binding the reporter molecule located in the conjugate pad of a support as defined in section b) below; and
b. A support suitable for lateral flow that comprises:
   b1. A backing card; and
   b2. A sample pad, a membrane and a wicking pad, all of them on top of the backing pad, wherein the membrane is located after the sample pad so that the sample flows from the sample pad to the membrane, wherein the membrane comprises a test line which in turn comprises molecules immobilized in the test line for binding to the capture tag of the polynucleotide sequence, wherein the wicking pad is located at the end of the membrane (see Figure 2), and
   wherein the system further comprises a conjugate pad between the sample pad and the membrane, which in turn comprises a reporter molecule capable of binding one of the capture tags of the polynucleotide sequence.

In a preferred embodiment of the fourth aspect of the invention, the membrane further comprises a control line.

In a preferred embodiment of the second or fourth aspect of the invention, the system comprises a support to incubate a biological sample and the polynucleotide sequence.

A fifth aspect of the invention provides for the use of the system of the second or fourth aspect of the invention, for detecting the presence of coronavirus, in particular SARS CoV-2 Coronavirus, in a biological sample.

### Brief description of the figures

**Figure 1****.** Scheme 1 of the Lateral Flow Probe Chromatography (LFPC), where the polynucleotide probe is functionalized on the surface of the reporter molecule by chemical coupling.
**Figure 2****.** Scheme 2 of the Lateral Flow Probe Chromatography (LFPC), where the polynucleotide probe is modified at both ends with capture tags. In this system the reporter is functionalized with a recognition molecule that binds the polynucleotide.

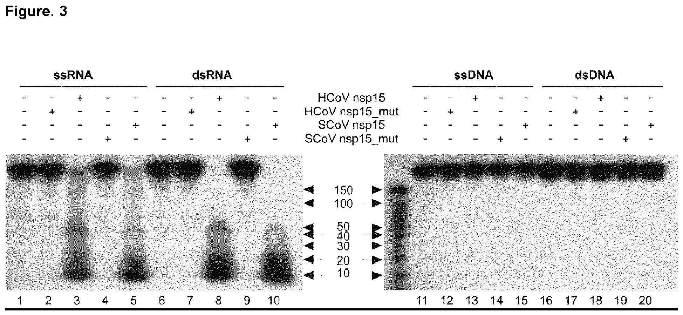

**Figure 3** (obtained from Konstantin A. Ivanov et al. PNAS 2004;101:34:12694-12699) shows RNA-specific endonuclease activity of SARS-CoV and HCoV-229E nsp15. ss and ds versions of 5'-[32P] RNA and DNA substrates, respectively, of 1 kb(p) were incubated for 20 min at 37°C without protein (lanes 1, 6, 11, and 16), with HCoV-229E MBP-nsp15_H6360A (HCoV nsp15_mut; lanes 2, 7, 12, and 17), with HCoV-229E MBP-nsp15 (HCoV nsp15; lanes 3, 8, 13, and 18), with SARS-CoV MBP-nsp15_H6678A (SCoV nsp15_mut; lanes 4, 9, 14, and 19) or with SARS-CoV MBP-nsp15 (SCoV nsp15; lanes 5, 10, 15, and 20). Reaction products and RNA size markers were separated in an 8% polyacrylamide gel containing 0.1% SDS.

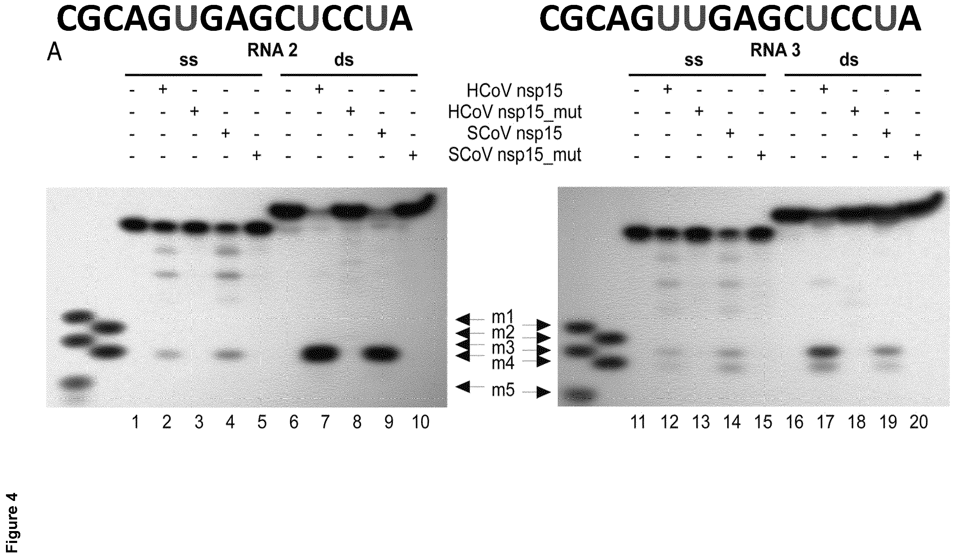

**Figure 4** (also obtained from Konstantin A. Ivanov et al. PNAS 2004;101:34:12694-12699) shows RNA-specific endonuclease activity of SARS-CoV and HCoV-229E nsp15. ss and ds versions of 5'-[32P] RNA and DNA substrates, respectively, of 1 kb(p) were incubated for 20 min at 37°C without protein (lanes 1, 6, 11, and 16), with HCoV-229E MBP-nsp15_H6360A (HCoV nsp15_mut; lanes 2, 7, 12, and 17), with HCoV-229E MBP-nsp15 (HCoV nsp15; lanes 3, 8, 13, and 18), with SARS-CoV MBP-nsp15_H6678A (SCoV nsp15_mut; lanes 4, 9, 14, and 19) or with SARS-CoV MBP-nsp15 (SCoV nsp15; lanes 5, 10, 15, and 20). Reaction products and RNA size markers were separated in an 8% polyacrylamide gel containing 0.1% SDS.
**Figure 5** (also obtained from Konstantin A. Ivanov et al. PNAS 2004;101:34:12694-12699) shows the importance of different divalent cations, in particular Mn2+, in the activity of the Nsp15 Endonuclease.
**Figure 6****.** Validation of saliva samples in healthy donors.

### Detailed description of the invention

### Definitions

In the context of the present invention "polynucleotide" is understood as an oligonucleotide substrate (single or double stranded) susceptible of being cleaved by the nsp15 endonuclease derived from coronovirus. The polynucleotide composition consists of natural nucleic acids (RNA), chemically modified nucleic acids or a combination of both.
In the context of the present invention "capture tag" is understood as the modification at the end of the "polynucleotide" that allows binding with the capture molecule and/or the reporter molecule. This binding is carried out by interaction of antigen-antibody or a similar biorecognition process.
In the context of the present invention "capture molecule" is understood as recognition molecule (e.g. biotin antibody) immobilized in the detection line of the lateral flow assay, with the capability to bind one of the ends of the polynucleotide probe that was previously modified with a capture tag (e.g. biotin).
In the context of the present invention "reporter molecule" is understood as a) the molecule that provides the readout of the system and consists of a latex/gold nanoparticle, carbon particle or a similar colorimetric molecule, functionalized with a capture molecule (e.g. antibody) that recognizes the polynucleotide probe via a capture tag. b) The reporter molecule consists of a latex/gold nanoparticle that is functionalized with the polynucleotide probe, at one of its ends by coupling chemistry (e.g. amine), while the other end is modified with a capture tag (e.g. biotin).
In the context of the present invention "lateral flow assay" is understood as a simple methodology to detect, in a specific manner, the presence or absence of the nsp15 endonuclease in a given matrix sample. This type of assay eliminates the need for specialized and costly equipment used in conventional laboratories. Lateral flow technology is used as point of care tool (fast and in situ), in a large range of applications, from the widely used home pregnancy test to more specialized tests for clinical, food safety, environmental and industrial applications.
In the context of the present invention "a backing card" is understood as the material where all the membranes (sample, conjugate, membrane and wicking pads) are mounted and connected to each other. At the same time, the backing card provides better stability and handling to the lateral flow device.
In the context of the present invention "a sample pad" is understood as the element of the device where the sample is introduced or deposited and acts as a sponge that holds the sample fluid. Subsequently, the sample fluid is transported to the membrane by capillary action.
In the context of the present invention "the conjugate pad" is understood as the part of the device where the reporter molecules are dispensed. The reporter molecules are immediately released from the conjugate pad upon contact with the sample fluid.
In the context of the present invention "a membrane" is understood as the material (e.g. nitrocellulose) that transports the sample and provides support to the capture molecules (antibodies, aptamers etc.) and allows or enhances their binding capabilities.
In the context of the present invention "a wicking pad" is understood as element in the system that retains all the assay material acting as a waste container.
In the context of the present invention "biological sample" is understood, as used in this invention, to be any sample of, relating to, caused by, or affecting life or living organisms, biological processes, such as growth and digestion. Examples of a biological sample may include, but are not limited to cell culture, cell culture supernatant, saliva, tears, urine, blood, serum, plasma, sweat, vaginal fluids, semen, feces, mucous, breast milk, ascites, lymph, pleural effusion, synovial fluid, bone marrow, cerebro-spinal fluid, and washings from bodily cavities (e.g., bronchial lavage), hair, tissue, bones, or teeth. Preferably, the biological sample is a liquid sample. Liquid means a state of matter with definite volume but no definite shape at 25° C., like water. More preferably, the biological samle is saliva.
In the context of the present invention "test sample" refers to any material being assayed for nuclease activity or enzymatic activity and in certain embodiments, will be a liquid.

### Description

Rapid identification of coronavirus, in particular SARS CoV-2 Coronavirus, is now possible taking advantage of the specific nuclease activity exerted by this virus, the activity of nuclease nsp15. The identification and/or design of specific nucleic acid probes, as those identified in table 1 above, that are degraded by the target virus but not by the rest of the microorganisms/cell types is thus critical for this purpose.

In this sense, we have developed specific LFPC strips that can detect non-degraded polynucleotide probes in a given sample. Accordingly, the presence of a polynucleotide probe will give rise to a colored test line. On the other hand, if the specific nuclease nsp15 is present in the sample it will eventually produce a complete degradation of the polynucleotide probe, and consequently, no test line will be seen.

Such system provides for a rapid and sensitive molecular approach for coronavirus, in particular SARS CoV-2 Coronavirus. For that purpose, the present invention provides for an *in vitro* use of a polynucleotide sequence susceptible of being cleaved by the specific nuclease nsp15 derived from coronavirus, in particular SARS CoV-2 Coronavirus, in a lateral flow assay.

In certain embodiments, the said polynucleotide sequence susceptible of being cleaved by the specific nuclease nsp15 derived from coronavirus, in particular SARS CoV-2 Coronavirus, is a short oligonucleotide probe (substrate) composed of nucleic acids and flanked with a capture tag on one end and i) a reporter molecule or ii) a further capture tag capable of binding the reporter molecule located in the conjugate pad of a support of a lateral flow device, on the other end. Upon cleavage of the probes by the nuclease (ribonuclease) in a sample, in particular in a biological sample, the capture tag is released away from the reporter molecule indicating the presence of said nuclease in a biological sample in a lateral flow device. The probes can thus be used to detect the presence of coronavirus, in particular SARS CoV-2 Coronavirus, in biological samples such as body fluids (blood serum, urine, CSF, semen, sputum, saliva, etc.) and solid tissue homogenized, cell cultures, food, environmental and industrial samples and also *in vivo.*

The present invention thus relates to a method for detecting the specific nuclease nsp15 activity in a test sample (biological sample), comprising: 1) incubating a synthetic Substrate or mixture of Substrates (from hereinafter "Substrate" or "substrate reagent") in the sample, for a time sufficient for cleavage of the Substrate(s) by the nuclease enzyme, wherein the Substrate(s) comprises a single-stranded (or double-stranded) nucleic acid molecule containing at least one ribonucleotide or deoxyribonucleotide or chemically modified nucleotide residue at an internal position that functions as a nuclease (ribonuclease) cleavage site (and in certain embodiments a 2'-O-methyl modified nucleotide that renders the oligonucleotide resistant to degradation by non-targeted nuclease activity), a capture tag on one end and i) a reporter molecule or ii) a further capture tag capable of binding the reporter molecule located in the conjugate pad of a support of a lateral flow device, on the other end, and 2) detecting the result, preferably visually, in particular whether the substrate has been cleaved or not by a test sample, by using a lateral flow device. Specific lateral flow devices useful to practice the present invention are illustrated through-out the description.

The Substrate oligonucleotide used to practice the present invention thus comprises a capture tag at one end and i) a reporter molecule or ii) a further capture tag capable of binding the reporter molecule located in the conjugate pad of the support of the lateral flow device, at the other end. Cleavage of the Substrate with the specific nuclease nsp15 enzyme leads to strand cleavage and physical separation of the capture group from the reporter group. Separation of reporter and capture eliminates the possibility of detecting a signal in the test line of the lateral flow device thus resulting in the detection of nuclease activity in the sample. The absence of the resulting signal can be detected by direct visual inspection of the lateral flow device.

Thus, the present invention is directed to the *in vitro* use of a polynucleotide sequence, or Substrate, susceptible of being cleaved by the specific nuclease nsp15 derived from coronavirus, in particular SARS CoV-2 Coronavirus, wherein the polynucleotide sequence is characterized by comprising a capture tag and a reporter molecule at each end of the sequence, for detecting the presence of coronavirus, in particular SARS CoV-2 Coronavirus,in a biological sample by using a lateral flow device.

In one embodiment, the Substrate used to practice the method of the invention to identify the presence of coronavirus, in particular SARS CoV-2 Coronavirus, is an oligonucleotide comprising ribonucleotide residues. The Substrate can also be a chimeric oligonucleotide comprising RNase-cleavable, e.g., RNA, residues, or modified RNase-resistant RNA residues. Preferably, Substrate composition is such that cleavage is a ribonuclease-specific event and that cleavage by enzymes that are strictly deoxyribonucleases does not occur.

Preferably, the Substrate is a chimeric oligonucleotide comprising ribonucleotide residue(s) and modified ribonucleotide residue(s). In one embodiment, the Substrate is a chimeric oligonucleotide comprising ribonucleotide residues and 2'-O-methyl ribonucleotide residues. In one embodiment, the synthetic Substrate is a chimeric oligonucleotide comprising 2'-O-methyl ribonucleotide residues and one or more residues of ribonucleotides such as uridine.

In another embodiment, the oligonucleotide of the Substrate is selected from one or more of the oligos listed in table 1.

Preferably, the oligonucleotide of the Substrate is selected from the list consisting of SEQ ID No 2.

Preferably, the oligonucleotide of the Substrate is selected from the list consisting of SEQ ID No 3.

Preferably, the oligonucleotide of the Substrate is selected from the list consisting of SEQ ID No 4.

Preferably, the oligonucleotide of the Substrate is selected from the list consisting of SEQ ID No 5.

It is noted that the method of the invention procees in two steps. First, the test sample is mixed with the Substrate reagent and incubated. the Substrate/s can be mixed alone with the test sample or will be mixed with an appropriate buffer. Second, there is a, preferably visual, detection of the result by using a lateral flow device. As illustrated in the examples and figures of the present invention, the presence of a control line and the lack of presence of a test line in the lateral flow device, indicates whether that nuclease activity is present in the test sample. The methods provide that this step can be done with unassisted visual inspection.

While the methods of the invention, when practiced by using a lateral flow device, can be practiced without the use of a control line in the lateral flow device, in certain embodiments of the invention it is desirable to include said control lines, such as where the control line is used as a negative control, the control sample, in some embodiments, contains no detectable nuclease activity. Thus, the present invention further provides a method, in accordance with the previous method of the invention, for detecting nuclease activity in a test sample, comprising: (a) contacting the test sample with a substrate, thereby creating a test reaction mixture, wherein the substrate comprises a nucleic acid molecule comprising (i) a cleavage domain comprising a single-stranded region; (ii) a capture tag at one side of the nucleic acid molecule; and (iii) a i) a reporter molecule or ii) a further capture molecule capable of binding the reporter molecule located in the conjugate pad of the support of the lateral flow device, at the other side of the nucleic acid molecule; (b) incubating the test reaction mixture for a time sufficient for cleavage of the substrate by a nuclease in the sample; and (c) determining whether a detectable signal is present in the test line of the lateral flow device, wherein presence of the said signal and the presence of a signal in the control line indicates that the sample fails to contain nuclease activity and absence of said signal and presence of a signal in the control line indicates that the sample contains nuclease activity.

The methods of the invention can further entail contacting the test sample with a buffer before or during step (a).

The present invention further provides compositions and kits for practicing the present methods. Thus, in certain embodiments, the present invention provides a kit comprising: (a) in one container, a substrate, the substrate comprising a nucleic acid molecule comprising a single stranded region, the single-stranded region comprising: (i) a cleavage domain; (ii) a capture tag on one side of the nucleic acid molecule; (iii) a i) a reporter molecule or ii) a further capture tag capable of binding the reporter molecule located in the conjugate pad of the support of the lateral flow device, on the other side of the nucleic acid molecule; and (b) preferably a lateral flow device. These kits are further described at the end of the description in the present specification, in the section entitled "Lateral flow devices and tests".

With respect to the capture tags, any compound that can be captured in a lateral flow assay and that can be bound to the single stranded region can be used in the methods and compositions of the invention. Numerous compounds are capable of that, such as BIOTIN, FITC, FAM, DIGOXIGENIN, DNP (DINITROPHENYL), TEXAS RED, TAMRA or any other small antigen.

In certain embodiments, the capture molecule is an antibody or other recognition molecules such as fab-fragments, nanobodies, aptamers, etc.

With respect to the reporter group, numerous compounds are capable of that, such as latex or gold nanoparticles, carbon particles, or other colorimetric reporter molecules.

The nucleic acids of the invention, including those for use as substrates in the methods of the invention, are at least 1, 2, or 3 nucleotides in length, such as 5-30 nucleotides in length. In certain specific embodiments, the nucleic acids of the invention are 5-20, 5-15, 5-10, 7-20, 7-15, 7-10, 5-50 or 10-50 nucleotides in length.

In certain embodiments, the capture tag of the nucleic acids of the invention is 5' to the cleavage domain and the reporter molecule is 3'to the cleavage domain. In a specific embodiment, the capture tag is at the 5' terminus of the substrate. In another specific embodiment, the reporter molecule is at the 3' terminus of the substrate.

In certain embodiments, the reporter molecule of the nucleic acids of the invention is 5' to the cleavage domain and the capture molecule is 3' to the cleavage domain. In a specific embodiment, the reporter molecule is at the 5' terminus of the substrate. In another specific embodiment, the capture tag is at the 3' terminus of the substrate.

In one embodiment of the invention, a nucleic acid of the invention comprising the formula: 5'-N1-n-N2 -3', wherein: (a) "N1" represents zero to five 2'-modified ribonucleotide residues; (b) "N2" represents one to five 2'-modified ribonucleotide residues; and (c) "n" represents one to ten, such as four to ten unmodified ribonucleotide residues, in particular U residues. In a certain specific embodiment, "N1" represents one to five 2'-modified ribonucleotide residues. In certain modes of the embodiment, the capture molecule or the reporter molecule is attached to the 5'-terminal 2'-modified ribonucleotide residue of N1.

In the nucleic acids of the invention, including nucleic acids with the formula: 5'-N1-n-N2-3', the capture tag can be 5' to the cleavage domain and the reporter molecule is 3' to the cleavage domain; alternatively, the reporter molecule is 5' to the cleavage domain and the capture molecule is 3' to the cleavage domain.

With respect to the kits of the invention, in addition to comprising a nucleic acid of the invention, the kits can further comprise one or more of the following: a nuclease; ribonuclease-free water, a buffer, and nuclease-free laboratory plastic ware, in particular the ribonuclease-free water and/or the buffer can be saturated with the divalent cation Mn²⁺.

### Substrate Oligonucleotides

As already stated, the oligonucleotide Substrates used to practice the present invention are substrates for nuclease nsp15 activity. Such substrate oligonucleotides comprise: 1) one or more nuclease-cleavable bases by nuclease nsp15, 2) a capture tag and a i) a reporter molecule or ii) a further capture tag capable of binding the reporter molecule located in the conjugate pad of the support of a lateral flow device, and 3) may optionally contain RNase-resistant modified RNA bases.

In certain embodiments, the substrate oligonucleotide probes are single-stranded or double-stranded oligoribonucleotides. In certain embodiments, the oligonucleotide probes are composed of modified oligoribonucleotides. The term "modified" encompasses nucleotides with a covalently modified base and/or sugar. For example, modified nucleotides include nucleotides having sugars, which are covalently attached to low molecular weight organic groups other than a hydroxyl group at the 3' position and other than a phosphate group at the 5' position. Thus, modified nucleotides may also include 2' substituted sugars such as 2'-O-methyl-; 2'-O-alkyl; 2'-O-allyl; 2'-S-alkyl; 2'-S-allyl; 2'-fluoro-; 2'-halo or 2'-azido-ribose, carbocyclic sugar analogues a-anomeric sugars; epimeric sugars such as arabinose, xyloses or lyxoses, pyranose sugars, furanose sugars, and sedoheptulose. In certain embodiments, the Substrate includes, but is not limited to, 2'-O-methyl RNA, 2'-methoxyethoxy RNA, 2'-O-allyl RNA, 2'-O-pentyl RNA, and 2'-O-butyl RNA.

Modified nucleotides are known in the art and include, by example and not by way of limitation, alkylated purines and/or pyrimidines; acylated purines and/or pyrimidines; or other heterocycles. These classes of pyrimidines and purines are known in the art and include, pseudoisocytosine; N4, N4-ethanocytosine; 8-hydroxy-N6-methyladenine; 4-acetylcytosine, 5-(carboxyhydroxylmethyl) uracil; 5-fluorouracil; 5-bromouracil; 5-carboxymethylaminomethyl-2-thiouracil; 5-carboxymethylaminomethyl uracil; dihydrouracil; inosine; N6-isopentyl-adenine; 1-methyladenine; 1-methylpseudouracil; 1-methylguanine; 2,2-dimethylguanine; 2-methyladenine; 2-methylguanine; 3-methylcytosine; 5-methylcytosine; N6-methyladenine; 7-methylguanine; 5-methylaminomethyl uracil; 5-methoxy amino methyl-2-thiouracil; β-D-mannosylqueosine; 5-methoxycarbonylmethyluracil; 5-methoxyuracil; 2-methylthio-N6-isopentenyladenine; uracil-5-oxyacetic acid methyl ester; psueouracil; 2-thiocytosine; 5-methyl-2 thiouracil, 2-thiouracil; 4-thiouracil; 5-methyluracil; N-uracil-5-oxyacetic acid methylester; uracil 5-oxyacetic acid; queosine; 2-thiocytosine; 5-propyluracil; 5-propylcytosine; 5-ethyluracil; 5-ethylcytosine; 5-butyluracil; 5-pentyluracil; 5-pentylcytosine; and 2,6,-diaminopurine; methylpsuedouracil; 1-methylguanine; 1-methylcytosine.

The oligonucleotides used to practice the present invention may be synthesized using conventional phosphodiester linked nucleotides and synthesized using standard solid or solution phase synthesis techniques, which are known in the art. Linkages between nucleotides may use alternative linking molecules. For example, linking groups of the formula P(O)S, (thioate); P(S)S, (dithioate); P(O)NR2; P(O)R'; P(O)OR6; CO; or CONR'2 wherein R is H (or a salt) or alkyl (1-12C) and R6 is alkyl (1-9C) is joined to adjacent nucleotides through -O- or -S-.

In certain embodiments of the present invention, the oligonucleotides have additional modifications, such as a 2'-O-methyl modification of the pyrimidines, in particular in the uridines.

The oligonucleotides are short, such as between 1-30 nucleotides in length (or any value in between). In certain embodiments, that oligonucleotide is between 10-15 nucleotides in length. In certain embodiments, that oligonucleotide is between 11-13 nucleotides in length. In general, shorter sequences will give better signal to noise ratios than longer probes and will therefore be more sensitive. However, in certain embodiments, shorter probes might not be the best substrate for the nuclease, so some degree of empiric optimization for length is needed. In certain embodiments, the oligonucleotide comprises 0-50% purines (or any value in between). In certain embodiments the oligonucleotide comprises 100% pyrimidines.

### Substrate Synthesis

Synthesis of the nucleic acid Substrates used to practice the present invention can be performed, but without being limited to, using solid-phase phosphoramidite chemistry (U.S. Pat. No. 6,773,885) with automated synthesizers, although other methods of nucleic acid synthesis (e.g., the H-phosphonate method) may be used. Chemical synthesis of nucleic acids allows for the production of various forms of the nucleic acids with modified linkages, chimeric compositions, and nonstandard bases or modifying groups attached in chosen places throughout the nucleic acid's entire length.

### Detection Methods

The present invention provides a method for detecting the specific nuclease nsp15 derived from coronavirus, in particular SARS CoV-2 Coronavirus, in a sample *in vitro* by using a lateral flow device. The method of the invention proceeds in the following steps: combine "test or biological sample" with Substrate(s), to produce a mixture, the mixture being the Assay Mix, incubate, and detect whether a signal is produced in a test line of a lateral flow device, "Test sample" refers to any material being assayed for nuclease activity and in certain embodiments, will be a liquid. Solids can be indirectly tested for the presence of nuclease contamination by washing or immersion in solvent, e.g., water, followed by assay of the solvent.

For example, one can contact a sample with an oligonucleotide probe as described herein and detect the presence of nuclease nsp15 activity derived from coronavirus, in particular SARS CoV-2 Coronavirus, using a lateral flow device.

In certain embodiments, the probes and molecules of the present invention are also useful to detect viral contamination in settings such as research laboratories.

Each of the steps of the method will be further illustrated, but without being limited to, as follows below:
**1. Assay Mix.** The Substrate/s is mixed and incubated with the test sample. This mixture constitutes the Assay Mix. Ideally, the Assay Mix is a small volume, from about 1 µl to about 10 ml, or, from about 10 to 100 µl The precise volume of the Assay Mix will vary with the nature of the test sample and the detection method. Optionally, a buffer can be added to the Assay Mix. Nucleases, including some ribonucleases, require the presence of divalent cations for maximum activity and providing an optimized buffered solution can increase the reaction rate and thereby increase assay sensitivity. Buffers of different composition can be used, as described in U.S. Pat. No. 6,773,885. The divalent cations shall be preferably selected from Mn²⁺ cations.
**2. Incubation**. The Assay Mix (e.g., the test sample plus Substrate/s) is incubated. Incubation time and conditions can vary, but it should take approximately 30 minutes or less depending upon the sensitivity required. Incubation temperature can generally vary from room temperature to 37 degree C. but may be adjusted to the optimal temperature of a specific nuclease suspected as being present as a contaminant.
**3. Signal Detection**.
   For signal detection two different systems may be employed according to the present invention:
   A first system comprises:
      a. The polynucleotide sequence/s as defined in the first aspect of the invention;
      b. A support suitable for lateral flow that comprises:
         b1. A backing card; and
         b2. A sample pad, a membrane and a wicking pad, all of them on top of the backing pad, wherein the membrane is located after the sample pad so that the sample flows from the sample pad to the membrane, wherein the membrane comprises a test line which in turn comprises molecules immobilized in the test line for capturing the capture molecule of the polynucleotide sequence, wherein the wicking pad is located at the end of the membrane.
         Assay Mix. The sample is incubated with the polynucleotide sequence as defined in the first aspect of the invention, in this system latex/gold nanoparticles can be use to functionalized the polynucleotide sequence as defined in the first aspect of the invention at one end of each of these moieties. The other end of the moieties can be modified with a capture tag. After incubation at desired temperature and time to allow efficient degradation of the polynucleotide, the assay mix is dispensed on the sample pad of the lateral flow device. Subsequently, the sample fluid is transported to the membrane and the capture tags can be detected by the capture molecules at the test line/s. If the nuclease activity is present, the capture molecule will detect a fragment of the polynucleotide that was functionalized with the capture tag, with no visible signal. If the nuclease activity is absent, the capture molecule will capture the biotinylated polynucleotide along with the reporter molecule, and thus will provide a readout signal that can be evaluated by naked eye. A schematic representation of this system is provided in Figure 1.
   A second system comprises:
      a. The polynucleotide sequence/s as defined in the third aspect of the invention conjugated in both ends to capture molecules, wherein one of the capture molecules of the moiety (the polynucleotide sequence) is capable of binding the reporter molecule located in the conjugate pad of the support; and
      b. A support suitable for lateral flow that comprises:
         b1. A backing card; and
         b2. A sample pad, a conjugate pad, a membrane and a wicking pad, all of them on top of the backing pad, wherein the membrane is located after the sample pad so that the sample flows from the sample pad to the membrane, wherein the membrane comprises a test line which in turn comprises molecules immobilized in the test line for capturing the capture molecule of the polynucleotide sequence, wherein the wicking pad is located at the end of the membrane.
            and wherein the system further comprises a conjugate pad between the sample pad and the membrane which in turn comprises a reporter molecule.
      Assay Mix. In this system, the sample is incubated with the polynucleotide probe, wherein the moieties are modified at both ends with two different capture tags. After incubation at desired temperature and time to allow efficient degradation of the polynucleotide, the assay mix is dispensed on the sample pad of the lateral flow device. Subsequently, the sample fluid is transported to the conjugate pad, where the reporter molecule was previously dispensed. This reporter molecule was modified with a capture molecule (e.g. anti-digoxigenin) that recognizes one of the capture tags of the polynucleotide (e.g. digoxigenin). Next, the assay mix and the reporter molecule are transported to the membrane where the test line/s is located and the capture molecule for detecting the moieties is immobilized. In this regard, one test line (the nuclease test line) shall be used to practice the method of the invention
**4. Visual Detection Method**. Following incubation, the Assay Mix is used in each of the systems described above as detailed as follows.

Regarding the first system, the assay mix used once incubated is placed in the sample pad of the lateral flow system. Next, the sample fluid is transported to the membrane and the capture molecule immobilized in the test line/s (e.g. anti-biotin) will recognize (each) of the capture tags located at one of the end of the polynucleotide probe. If the nuclease activity is present, the capture molecule will detect a fragment of the polynucleotide that was functionalized with the capture tag, with no visible signal. If the nuclease activity is absent, the capture molecule will capture the biotinylated polynucleotide along with the reporter molecule, and thus will provide a readout signal that can be evaluated by naked eye.

This system will provide a readout signal that can be evaluated by naked eye.

Regarding the second system, the assay mix used once incubated is placed in the sample pad of the lateral flow system and later mixed with the conjugate pad, where the reporter molecule was previously dispensed. Next, the sample fluid is transported to the membrane and the capture tag immobilized in the test line/s will recognize the capture tags located at one of the end of the polynucleotide probe. In this regard, one test line (the nuclease test line) shall be used to practice the method of the invention.

Therefore, an Assay Mix in which the Substrate remains intact will provide for a test signal and will visually appear. An Assay Mix in which the Substrate has been cleaved will not provide for a test signal and will visually appear clear or dark (no color). The visual detection method is primarily intended for use as a qualitative nuclease assay, with results being simply either "positive" or "negative".

The Assay Mix will ideally constitute a relatively small volume, for example 10µl to 1000µL although greater or lesser volumes can be employed. Small volumes allow for maintaining high concentrations of Substrate yet conserves use of Substrate. The visual detection Assay in one embodiment uses 50 pmoles of Substrate at a concentration of 0.5 µM in a 100 µl final volume Assay Mix. Lower concentration of Substrate (e.g., below 0.1 pM) will decrease assay sensitivity. Higher concentrations of Substrate (e.g., above 1 µM) will increase background and will unnecessarily consume Substrate.

Steps (mixing, incubating), can be performed in one tube. In one embodiment, the tube is a small, thin-walled, UV transparent micro tube, although tubes of other configuration may be used.

### Lateral flow devices and tests

The present invention further includes lateral flow devices and kits containing the same for detecting the specific nuclease nsp15 activity derived from coronavirus, in particular SARS CoV-2 Coronavirus, in a sample, comprising Substrate nucleic acid(s), and optionally instructions for use. Such kits may optionally contain one or more of: a positive control nuclease, DNase/RNase-free water, a buffer, and other reagents.

Lateral Flow tests, also known as Lateral Flow Immunochromatography (LFIC) tests or just Lateral Flow Immunoassays (LFI), are simple small devices intended to detect a target analyte in a given sample. These tests are simple, fast, and cheap and do not require specialized and costly equipment nor qualified personnel. Even though the LFIC technology was born in the early 80s, it is still going to be a major player in the next decades in the rapid test industry. In the beginning, LFIC tests were exclusively used in medical diagnostics (either for home testing, point of care testing, or laboratory use) but their presence in other areas are now very common. The first application was the well-known home pregnancy test. Although there are several commercially available semi-quantitative tests, LFIC tests are mainly qualitative tests and they just give a positive or negative result based on the presence or absence of the target analyte at a specific concentration.

The technology is based on a series of capillary beds with porous materials that has the capacity to transport liquids spontaneously. When the target analyte is big (e.g. a protein) a sandwich format is commonly used. Usually, the first element (the conjugate pad) has stored the so-called colloids, a dried format of bioactive particles (mainly gold nanoparticles or latex microparticles bound to a specific antibody against the target analyte) in a salt-sugar matrix. This conjugate pad usually acts also as the sample pad and it contains everything to facilitate the recognition of the target antigen by the colloid. Once the conjugate pad is soaked by the sample, the fluid dissolves the salt-sugar matrix and the colloids. Therefore, the sample and the colloid mix while they flow through the porous structure. In this way, the analyte binds to the colloid and the complex is transported into the nitrocellulose membrane. This material has one specific area called test line where a third molecule (usually another different antibody against the target analyte) has been immobilized. When the complex (colloid-antigen) reaches the test line it gets bound to this antibody and after a while, when more and more fluid has flown, colloids accumulate and the test line acquires color. Typically, there is also a control line (after the test line) that captures the colloids that have not bound to the test line. In the control line, a fourth molecule (could be an antibody against the Fc fraction of the first antibody) is adsorbed and its color acquisition ensures the test functionality. After passing these reaction zones the fluid enters the final porous material, the wick or sink that simply acts as a waste container.

To practice the present invention, a large variety of different lateral flow system devices could be used, however, two devices to which the present invention is not limited to are herein provided just for illustrative purposes:
A first lateral flow device comprising a support suitable for lateral flow, which in turn comprises:
   a1. A backing card; and
   a2. A sample pad, a membrane and a wicking pad, all of them on top of the backing pad, wherein the membrane is located after the sample pad so that the sample flows from the sample pad to the membrane, wherein the membrane comprises a test line which in turn comprises molecules immobilized in the test line for capturing the capture molecule of the polynucleotide sequence, and wherein the wicking pad is located at the end of the membrane.
A second lateral flow device comprising a support suitable for lateral flow, which in turn comprises:
   a1. A backing card; and
   a2. A sample pad, a membrane and a wicking pad, all of them on top of the backing pad, wherein the membrane is located after the sample pad so that the sample flows from the sample pad to the membrane, wherein the membrane comprises a test line which in turn comprises molecules immobilized in the test line for capturing the capture molecule of the polynucleotide sequence, wherein the wicking pad is located at the end of the membraner;
   and wherein the support further comprises a conjugate pad between the sample pad and the membrane, which in turn comprises a reporter molecule.

The following examples merely serve to illustrate the present invention and do not limit the same.

### Examples

### Example 1. Nsp15 endonuclease activity

As reflected in table 2 below, Nsp15 endonuclease activity is present in all known human coronavirus know thus, far, as reflected below (table 2).

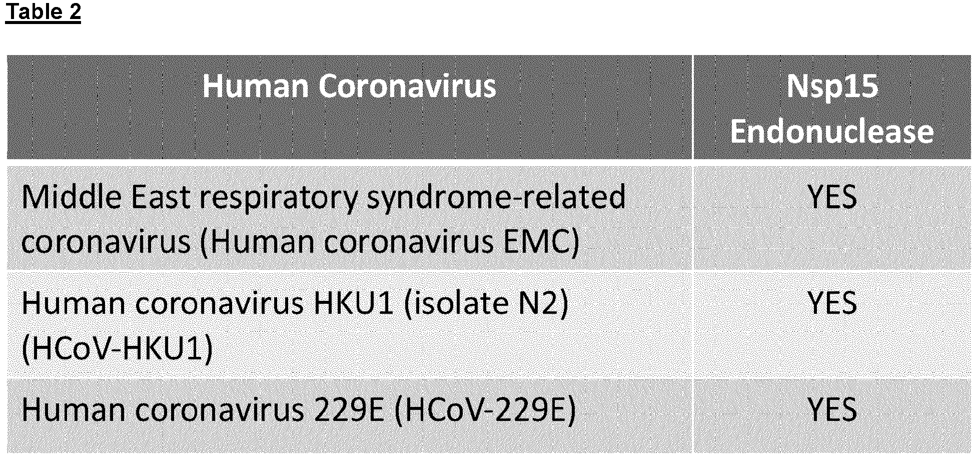

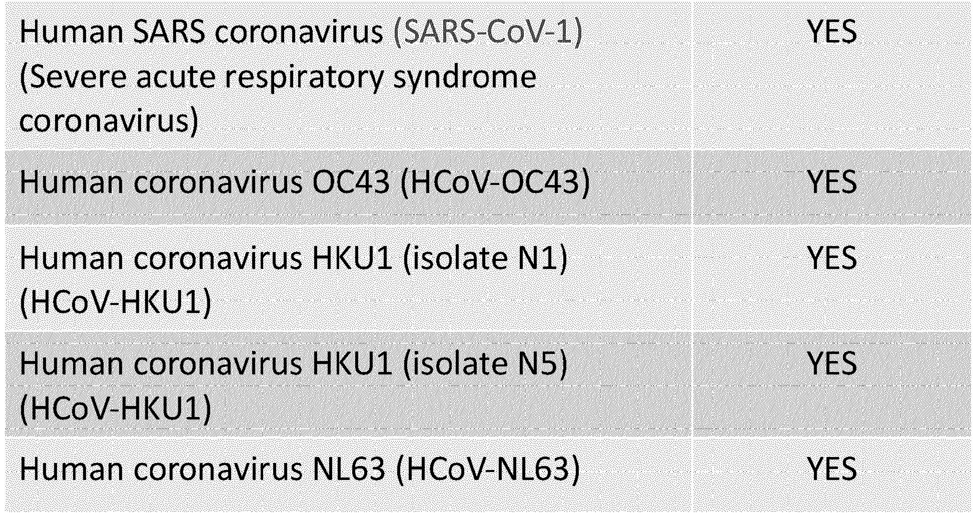

In addition, phylogenetic analyses of 15 HCoV whole genomes reveal that 2019-nCoV/SARS-CoV-2 shares the highest nucleotide sequence identity with SARS-CoV (79.7%). Specifically, the envelope and nucleocapsid proteins of 2019-nCoV/SARS-CoV-2 are two evolutionarily conserved regions, having the sequence identities of 96% and 89.6%, respectively, compared to SARS-CoV. Therefore, we can thus assume that, in view of such high nucleotide sequence identity with SARS-CoV, and in view of the fact that Nsp15 endonuclease activity is present in all known human coronavirus currently known, it is plausible that Human SARS coronavirus (SARS-CoV-2) CoV-19, has Nsp15 endonuclease activity.

It is important to note that All other RNA viruses do not produce Nsp15 Endonuclease

### Example 2. Specific Nsp15 endonuclease features

Figure 3 (obtained from Konstantin A. Ivanov et al. PNAS 2004;101:34:12694-12699) shows RNA-specific endonuclease activity of SARS-CoV and HCoV-229E nsp15. ss and ds versions of 5'-[32P] RNA and DNA substrates, respectively, of 1 kb(p) were incubated for 20 min at 37°C without protein (lanes 1, 6, 11, and 16), with HCoV-229E MBP-nsp15_H6360A (HCoV nsp15_mut; lanes 2, 7, 12, and 17), with HCoV-229E MBP-nsp15 (HCoV nsp15; lanes 3, 8, 13, and 18), with SARS-CoV MBP-nsp15_H6678A (SCoV nsp15_mut; lanes 4, 9, 14, and 19) or with SARS-CoV MBP-nsp15 (SCoV nsp15; lanes 5, 10, 15, and 20). Reaction products and RNA size markers were separated in an 8% polyacrylamide gel containing 0.1% SDS.

This figure shows the oligonucleotide substrate preference for the Nsp15 Endonuclease (ss are preferred).

Figure 4 (also obtained from Konstantin A. Ivanov et al. PNAS 2004;101:34:12694-12699) shows RNA-specific endonuclease activity of SARS-CoV and HCoV-229E nsp15. ss and ds versions of 5'-[32P] RNA and DNA substrates, respectively, of 1 kb(p) were incubated for 20 min at 37°C without protein (lanes 1, 6, 11, and 16), with HCoV-229E MBP-nsp15_H6360A (HCoV nsp15_mut; lanes 2, 7, 12, and 17), with HCoV-229E MBP-nsp15 (HCoV nsp15; lanes 3, 8, 13, and 18), with SARS-CoV MBP-nsp15_H6678A (SCoV nsp15_mut; lanes 4, 9, 14, and 19) or with SARS-CoV MBP-nsp15 (SCoV nsp15; lanes 5, 10, 15, and 20). Reaction products and RNA size markers were separated in an 8% polyacrylamide gel containing 0.1% SDS.

This figure shows the oligonucleotide substrate preference for the Nsp15 Endonuclease, and the cleavage sites for both oligos illustrated therein.

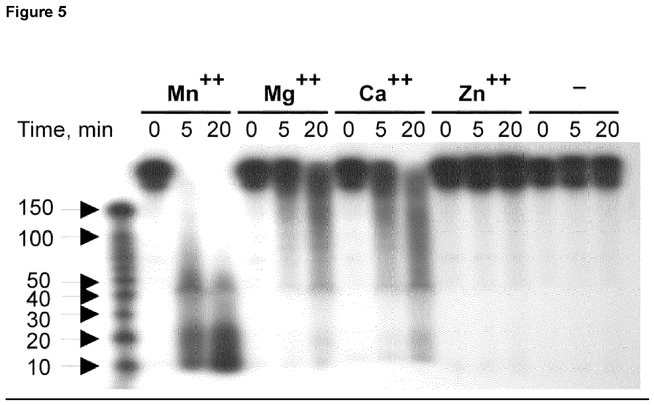

Lastly, figure 5 (also obtained from Konstantin A. Ivanov et al. PNAS 2004;101:34:12694-12699) shows the importance of the divalent cation Mn2+ in the activity of the Nsp15 Endonuclease.

Based on these results, within the context of the present invention, we can thus conclude, that a specific probe for Coronavirus with high specificity can be identified on the basis of the following criteria:
- Single Uridine degradation (unique feature).
- Mn⁺² favors nuclease activity (uncommon cofactor for other nucleases).
- Design of chemically modified oligos to avoid unspecific degradation by other viruses.

### Example 3.

As already reflected in example 1, there is a substantial body of evidence showing that coronaviruses encode several unusual RNA-processing enzymes, including an RNA endoribonuclease, namely Nsp15. This endonuclease it is very well characterized and possesses several unique features that can be successfully exploit within the context of the present invention: i) Nsp15 preferentially cleaves 3' of uridines (U) in a sequence of nucleotides; and ii) the nuclease activity is preferentially stimulated by Mn 2+ and not by other divalent cations that are most common to the majority of endonucleases (such as Mg2+, Ca2+). These two features are unique in the realm of nucleases and represent a real opportunity to design specific substrates with high sensitivity and specificity for the coronavirus. Based on these data, screening of clinical samples infected with coronavirus and non-infected (as controls) will be performed..

### 3.1. Description of the clinical samples (specimens): origin, size and type

Clinical samples will be provided by the University Hospital Cruces (Bilbao, Spain) and Hospital Txagorritxu in Vitoria-Gasteiz (Spain), one of the epicenters of the coronavirus infection outbreak is Spain. Relevant clinical samples would include those samples that have the greatest virus yield, such as respiratory samples (saliva, sputum, endotracheal aspirates, bronchoalveolar lavage) and possible other specimens could be included such as stool and blood. Nevertheless, the ideal type of sample would be saliva, that has been recently proven to have high enough viral load to allow early detection of SARS-CoV 19. At the same time, saliva is the safer alternative to the other types of samples, minimizing the distance and exposure of the healthcare giver to the infected patient.

### 3.2. Sample transportation and handling.

Transport of coronavirus infected specimens from Bilbao and Vitoria-Gasteiz to the Applicant's laboratories in San Sebastian (Spain) will comply with applicable national regulations. Testing on clinical specimens from patients meeting the suspect case definition will be performed in appropriately equipped laboratories by the Applicant's staff that is trained in the relevant technical and safety procedures. National guidelines on laboratory biosafety will be followed in all circumstances. Specimen handling for testing will be performed in Applicant's ' BSL-2 laboratory, equipped with negative pressure system. Specimens to be screened for the virus endonuclease detection should reach the laboratory as soon as possible after collection. Correct handling of specimens during transportation is essential. Specimens which can be delivered promptly to the Applicant's laboratory can be stored and shipped at 2-8°C. When there is likely to be a delay in specimens reaching the laboratory, the use of a specimen transport medium (containing protease inhibitor cocktail) to preserve the integrity if nucleases is strongly recommended.

Specimens may be frozen to - 20°C or ideally -70°C and shipped on dry ice if further delays are expected. It is important to avoid repeated freezing and thawing of specimens.

### 3.3. Screening procedure.

All clinical specimens (infected and controls) of a given type (e.g. saliva) will be processed under the same conditions. Previously, Applicant's has developed several protocols of sample processing (endotracheal aspirates, blood and stool) in order to perform nuclease activity assay or lateral flow assay. These protocols can be readily adapted for saliva or sputum testing, for example. Once the clinical specimen is processed, it is further subjected to the first round of screening using a library consisting of 100 oligonucleotide probes available at the Applicant's facilities. These probes are designed and synthesized with several types of chemical modifications at the sugar ring, such as 2'-OMethyl and 2'-Fluoro, that impart resistance to endogenous (non-target nucleases) and specificity to target nucleases, depending on the sequence design and nuclease substrate preference. The screening for the identification of the candidate substrates for the target endonuclease (Nsp15) of coronavirus will be performed by carrying out nuclease activity assay for both, control and infected samples. Additionally, a control reaction in phosphate buffer (PBS) only will correct for the probe background. The nuclease activity assay is the modality of assessing the degradation of an oligonucleotide substrate (or probe) by a given nuclease by providing an easy read-out, where the fluorescence intensity of the reading event is proportional to the degradation efficiency of the nuclease. The substrate probes that show significant fold differences (> 2) between positive and negative samples will be considered for further refining of the design and optimization in a second round of screening.

### 3.4. Validation of the lead probe

Lead probes obtained, as described above, will be further validated in 300 clinical samples.

### 3.5. Validaton of the methodology

In-house validation of the methodology of the present invention consisting of analytical characterization and diagnostic assessment to evaluate the sensitivity and specificity of the method consists of two components: 1) the probes with the sample; 2) a generic lateral flow platform (strip) that allows reaction read-out. Briefly, the lead nucleic acid probe (substrate) will be synthesized with two capture tags Digoxigenin "Tag#1" and Biotin "Tag#2"). The Digoxigenin tag binds a reporter molecule (Latex particle) previously functionalized with anti-digoxigenin (Anti-Tag#1) and located in the conjugate pad of the strip. On the other hand, the Biotin tag (Tag#2) captures the nucleic acid probe in the test line of the strip. One great advantage of the Applicant's' probes is the flexibility and adaptability to any type of commercially available generic lateral flow system. Specifically, the probes can be easily synthesized with various tags detectable by anti-tag capture molecules available in the strip.

Upon cleavage of the nucleic acid probe by nucleases derived from a coronavirus infected sample, the Digoxigenin tag (Tag#1) is released from the reporter molecule, thus indicating the presence of Nsp15 endonuclease in the sample in a lateral flow device.

The positive result shows no line in the test line (T), as indicated in the figures. If the target endonuclease is absent, the detection complex remains uncleaved and is visualized as a black line in the strip test line (negative result).

### 3.6. Functionality test.

The strip performance will be tested with 200 coronavirus positive samples and 50 controls (non-infected). Samples will be processed using a buffer specifically optimized for the specimen (either saliva or sputum). Initially, the reaction will be carried out at 37°C and for 1h, similar to the fluorescence nuclease activity assay. Next, the reaction will be optimized to be performed at room temperature and for a maximum of 25 minutes. These parameters have been already optimized for other types of ribonucleases with consistent response in the lateral flow system. For this reason, we think is perfectly plausible that a total of min 25-30 min (15 minutes reaction+ 10 minutes LF running time) will be the actual working time window from sample to the end result for the test.

### 3.7. Sensitivity and specificity tests.

The concentration of the probe will be optimized to reach the desired sensitivity. It is noteworthy to mention that the lateral flow system is 300 x more sensitive than the fluorescence system, thus allowing the use of lower amount of probe per reaction. It is expected that the lateral flow system will be able to detect even a few number of Nsp15 nucleases, thus a lower viral load, which is of outermost importance for the early detection of asymptomatic patients. This can be rationalized through the prism of the nuclease enzymatic reaction on their substrates that acts as an intrinsic amplification module, where the limiting factor is the probe. Moreover, to increase the sensitivity and specificity, the reaction buffer will contain Mn2+, which is the preferred cation for the Nsp15 nuclease activity.

### Example 4

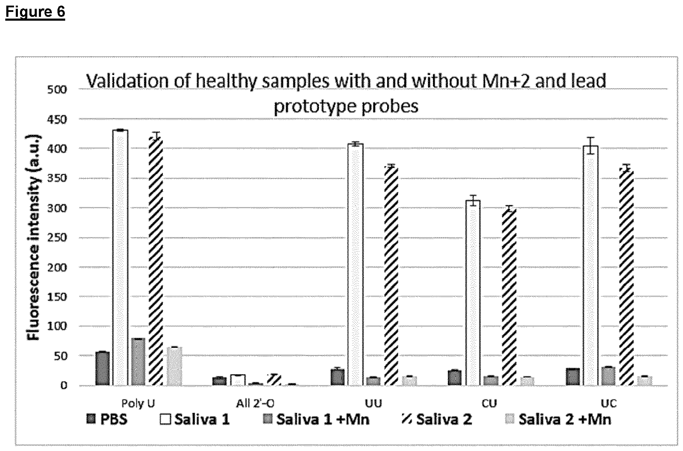

For this example, we tested two samples of saliva from healthy individuals to establish the background signals and the efficiency of Mn2+ to reduce unspecific nuclease activity. These results confirm the feasibility of detecting the nuclease activity derived from coronavirus Nsp15 endonuclease, which is a nuclease that specifically favors the cleavage of sequences containing Uridine RNA in the presence of Mn2+. The PBS samples show the background signal of the probes (dark gray), the All 2'-O-Methyl probe is used as negative control (resistant to degradation, white bars). The lead probes UU, CU, UC (SEQ ID NO 1 (positive control), SEQ ID NO 2 (UU), SEQ ID NO 33 (CU) y SEQ ID NO 34(UC)), that are flanked by four 2'-O-Methyl modified nucleosides at each end, have demonstrated a significant reduction of the unspecific degradation signal. These results suggest that Nsp15 endonuclease derived from Coronavirus has the capability to be differentiated from healthy samples based on its preference for single-or few-Uridine containing RNA substrates in the presence of Mn2+ (see figure 6).

## Claims

1. An *in vitro* use in a lateral flow assay of a polynucleotide sequence susceptible of being cleaved by the nsp15 endonuclease derived from coronovirus, wherein the polynucleotide sequence is **characterized by** comprising a capture tag and a reporter molecule at each end of the sequence, for detecting the presence of coronavirus, in particular SARS CoV-2 Coronavirus, in a biological sample.

2. The in vitro use of claim 1, wherein said polynucleotide sequence is selected from any of the sequences selected from the list consisting of SEQ ID No 1-34

3. The in vitro use of claim 1, wherein said polynucleotide sequence is selected from any of the sequences selected from the list consisting of SEQ ID No 1-34, wherein in one or more of these sequences mU has been replaced by _{LNA}U (Locked nucleic acid).

4. The in vitro use of any of claims 1 to 3, wherein the said capture tag is selected from the list consisting of **digoxigenin**:3-[(3*S*,5*R*,8*R*,9*S*,10*S*,12*R*,13*S*,14*S*,17*R*)-3,12,14-trihydroxy-10,13-dimethyl 1,2,3,4,5,6,7,8,9,11,12,15,16,17--tetradecahydrocyclopenta[a]phenanthren-17-yl]-2*H*-furan-5-one, **Biotin**: 5-[(3a*S*,4*S*,6a*R*)-2-oxohexahydro-1*H*-thieno[3,4-*d*]imidazol-4-yl]pentanoic acid, **ACA:** (6S,7S)-3-(acetoxymethyl)-7-amino-8-oxo-5-thia-1-azabicyclo[4.2.0]oct-2-ene-2-carboxylic acid **FAM:** 3',6'-dihydroxy-1-oxospiro[2-benzofuran-3,9'-xanthene]-5-carboxylic acid, **FITC:** 3',6'-dihydroxy-6-isothiocyanatospiro[2-benzofuran-3,9'-xanthene]-1-one, **Texas red:** 5-(Chlorosulfonyl)-2-(2,3,6,7,12,13,16,17-octahydro-1H,5H,11H,15H-pyrido[3,2,1-ij]quinolizino[1',9':6,7,8]chromeno [2,3-f]quinolin-4-ium-9-yl) Benzenesulfonate,and **TAMRA:** 2-[3-(dimethylamino)-6-dimethylazaniumylidenexanthen-9-yl]benzoate or any other small antigen.

5. The in vitro use of any of claims 1 to 4, wherein the reporter molecule is selected from the list consisting of latex and gold nanoparticles, carbon particles or any other colorimetric reporter molecule, wherein preferably the said reporter molecule is linked to the polynucleotide by using an antibody having specificity against the capture tag used to modify the polynucleotide probe.

6. A system that comprises:
a. The polynucleotide sequence as defined in any of claims 1 to 3;
b. A support suitable for lateral flow that comprises:
b1. A backing card; and
b2. A sample pad, a membrane and a wicking pad, all of them on top of the backing pad, wherein the membrane is located after the sample pad so that the sample flows from the sample pad to the membrane, wherein the membrane comprises a test line which in turn comprises molecules immobilized in the test line for capturing the capture tag of the polynucleotide sequence, and wherein the wicking pad is located at the end of the membrane.

7. The system of claim 6, wherein the membrane further comprises a control line.

8. An *in vitro* use in a lateral flow assay of a polynucleotide sequence susceptible of being cleaved by the nsp15 endonuclease derived from coronovirus, wherein the polynucleotide sequence is **characterized by** comprising two capture tags at each end of the sequence, wherein each capture tag is different from the other and wherein one of the capture tags is capable of binding a reporter molecule, for detecting the presence of coronavirus, in particular SARS CoV-2 Coronavirus, in a biological sample..

9. The in vitro use of claim 8, wherein said polynucleotide sequence is selected from any of the sequences selected from the list consisting of SEQ ID No 1-34.

10. The in vitro use of claim 8, wherein said polynucleotide sequence is selected from any of the sequences selected from the list consisting of SEQ ID No 1-34, wherein in one or more of these sequences mU has been replaced by _{LNA}U (Locked nucleic acid).

11. The in vitro use of any of claims 8 to 10, wherein the said capture tag is selected from the list consisting of **digoxigenin**:3-[(3*S*,5*R*,8*R*,9*S*,10*S*,12*R*,13*S*,14*S*,17*R*)-3,12,14-trihydroxy-10,13-dimethyl 1,2,3,4,5,6,7,8,9,11,12,15,16,17--tetradecahydrocyclopenta[a]phenanthren-17-yl]-2*H*-furan-5-one, **Biotin:** 5-[(3a*S*,4*S*,6a*R*)-2-oxohexahydro-1*H*-thieno[3,4-d]imidazol-4-yl]pentanoic acid, **ACA:** (6S,7S)-3-(acetoxymethyl)-7-amino-8-oxo-5-thia-1-azabicyclo[4.2.0]oct-2-ene-2-carboxylic acid **FAM:** 3',6'-dihydroxy-1-oxospiro[2-benzofuran-3,9'-xanthene]-5-carboxylic acid, **FITC:** 3',6'-dihydroxy-6-isothiocyanatospiro[2-benzofuran-3,9'-xanthene]-1-one, **Texas red:** 5-(Chlorosulfonyl)-2-(2,3,6,7,12,13,16,17-octahydro-1H,5H,11H,15H-pyrido[3,2,1-ij]quinolizino[1',9':6,7,8]chromeno [2,3-f]quinolin-4-ium-9-yl) Benzenesulfonate,and **TAMRA:** 2-[3-(dimethylamino)-6-dimethylazaniumylidenexanthen-9-yl]benzoate or any other small antigen.

12. The in vitro use of any of claims 8 to 11, wherein the reporter molecule is selected from the list consisting of latex and gold nanoparticles, carbon particles or any other colorimetric reporter molecule.

13. A system that comprises:
a. The polynucleotide sequence as defined in any of claims 8 to 10, wherein one of the capture molecules is capable of binding the reporter molecule located in the conjugate pad of a support as defined in section b) below; and
b. A support suitable for lateral flow that comprises:
b1. A backing card; and
b2. A sample pad, a membrane and a wicking pad, all of them on top of the backing pad, wherein the membrane is located after the sample pad so that the sample flows from the sample pad to the membrane, wherein the membrane comprises a test line which in turn comprises molecules immobilized in the test line for binding to the capture tag of the polynucleotide sequence, wherein the wicking pad is located at the end of the membrane, and
wherein the system further comprises a conjugate pad between the sample pad and the membrane, which in turn comprises a reporter molecule capable of binding one of the capture tags of the polynucleotide sequence.

14. The system of claim 13, wherein the membrane further comprises a control line.

15. In vitro use of the system of claim 6 or 13, for detecting the presence of coronavirus, in particular SARS CoV-2 Coronavirus, in a biological sample.
